# EUROPEAN PATENT SPECIFICATION

(11) **EP 4 188 545 B1**
(45) Date of publication and mention of the grant of the patent: **31.01.2024**
(21) Application number: 21761982.4
(22) Date of filing: 03.08.2021
(51) Int. Cl.: A61P 27/02, G01N 33/49

(54) **COMPOSITION FOR TREATING DAMAGED EPITHELIAL SURFACES AND METHOD OF PRODUCING SAME**
ZUSAMMENSETZUNG ZUR BEHANDLUNG VON BESCHÄDIGTEN EPITHELOBERFLÄCHEN UND VERFAHREN ZU DEREN HERSTELLUNG
COMPOSITION POUR LE TRAITEMENT DES SURFACES ÉPITHÉLIALES ENDOMMAGÉES ET SON PROCÉDÉ DE PRODUCTION

(30) Priority: 03.08.2020 EP 20189093
(43) Date of publication of application: 07.06.2023
(73) Proprietor: SerEye GmbH, 37079 Göttingen (DE)
(72) Inventor: STADLER, Herbert, 37127 Niemetal-Ellershausen (DE); SCHNEIDER, Stefan, 37085 Göttingen (DE); JOCHHEIM-RICHTER, Andrea, 61462 Königstein im Taunus (DE); SILLABER, Ingeborg, 80802 München (DE)
(74) Representative: Witthoff Jaekel Steinecke Patentanwälte PartG mbB
(86) International application number: PCT/EP2021/071638
(87) International publication number: WO 2022/029109

(56) References cited:
- US-A1- 2006 127 874
- US-A1- 2018 050 064
- SORENSEN O ET AL: "The human antibacterial cathelicidin, hCAP-18, is bound to lipoproteins in plasma", JOURNAL OF BIOLOGICAL CHEMISTRY, AMERICAN SOCIETY FOR BIOCHEMISTRY AND MOLECULAR BIOLOGY, vol. 274, no. 32, 6 August 1999 (1999-08-06), pages 22445-22451, XP002257545, ISSN: 0021-9258, DOI: 10.1074/JBC.274.32.22445
- BOZIC ET AL: "Viscosity of Plasma as a Key Factor in Assessment of Extracellular Vesicles by Light Scattering", CELLS, vol. 8, no. 9, 6 September 2019 (2019-09-06), page 1046, XP055768217, DOI: 10.3390/cells8091046

## Description

### FIELD OF THE INVENTION

The present invention generally relates to biologically active blood fractions that can be used for the treatment of damaged epithelial surfaces. In particular, the fractions are obtained via ultracentrifugation and subsequent separation from total serum or total plasma and are preferably used for treating damaged epithelial surfaces in connection with eye diseases. The invention also relates to corresponding compositions and eye drops as well as to a method of manufacture of eye drops.

### BACKGROUND OF THE INVENTION

Epithelial tissues are made up by epithelial cells and line the outer surfaces of organs and blood vessels throughout the body, as well as the inner surfaces of cavities in many internal organs. Thus, epithelia are the first line of defense between the human body and its environment. The stratified, non-keratinized epithelia of the cornea and conjunctiva are continuously exposed to allergens, debris, pathogens, desiccation, injury, and rubbing, wherein a variety of biologically active molecules comprised in the tear film are critical for the protection of these epithelia and the tear film plays a janitorial role by, *e.g.,* continuously washing noxious agents out of the ocular surface. If not enough tear film is produced or if there is not enough water, oil or mucus in the tear film, the eye is not lubricated properly leading to dry eyes and potentially damaging the epithelial cells on the surface of the cornea.

As regards alleviation of ophthalmic disorders coming along with dry eyes or epithelial damage, several wetting and lubricating agents are known, also referred to as artificial tears. Such artificial tears contain a range of water-soluble viscosity modifiers and wetting agents, to ensure that they cover the ocular surface and remain for prolonged periods, in order to make up for deficiencies in the quantity or quality of the natural tear film.

In each case, however, such artificial tears generally do not contribute directly to active healing of the underlying condition and frequent application of artificial tears containing preservatives to prevent contamination has been found to include both toxic and allergic reactions, especially in patients with sensitive eyes.

One particular approach that has been used with some success is to boost healing of damaged epithelia and to reduce inflammations locally by the administration of natural healing factors. For example, autologous serum or plasma eye drops have been developed, wherein the various proteins, enzymes, healing factors and growth factors naturally present in the serum/plasma assist healing of the underlying condition, supplementing the same compounds being delivered to the eye through the patient's bloodstream and natural tears.

However, the preparation of eye drops based on blood products is highly regulated, quite lengthy and depends on autologous blood donation which makes this process not feasible for the production of eye drops in industrial scale.

The present invention and its embodiments as characterized in the claims, taught in the description and illustrated in the Examples contributes to the solution of the above-mentioned problem.

### SUMMARY OF THE INVENTION

The present invention relies on the surprising finding that fractions of blood obtained after ultracentrifugation of serum are still biological active in having a healing effect on damaged epithelial cells and thus are useful in the treatment or amelioration of eye disease and disorders where such healing effect on damaged epithelial cells is beneficial. In particular, serum fractions are obtained that are substantially free of lipids and lipoproteins which increases their flow ability facilitating bottling of the fractions and sterile filtration for the removal of pathogens, in particular viruses. Similarly, blood plasma can be used as starting material for obtaining said biologically active fractions.

As mentioned above, blood serum and blood plasma are both useful for treating epithelial disorders and in particular for treating disorders associated with dry eye since serum and plasma are known to comprise a diverse range of proteins, enzymes, healing factors and growth factors supporting the healing of damaged cells.

There are several drawbacks associated with the production of products derived from blood. First of all, currently only products comprising autologous serum or plasma, *i.e.* serum/plasma derived from whole blood obtained from the patient to be treated, are available. However, processing the whole blood to produce serum or plasma free from blood cells is a lengthy process and it is hence only worthwhile when carried out on a significant quantity of blood at once, but of course the amount of blood that can be derived from the patient itself is limited to about 500 ml per blood donation. Furthermore, usually only patients between 18 and 68 years of age are allowed to donate blood and patients with iron deficiency or having cardiovascular diseases, severe diseases of the central nervous system or clinically relevant blood clotting disorders are not allowed to donate blood. Thus, in people with specific primary diseases involving inflammatory processes or also in elderly patients an autologous serum or plasma eye drop preparation might not be possible or even contra-productive. In addition, there is a high logistic effort until the patient received the product since for example precautions must be taken to ensure that the correct product is supplied to each patient and it has be ensured that the product remains fresh and sterile. Thus, only small batches can be produced.

The use of serum or plasma derived from whole blood obtained not only from the person to be treated, but from several donors entails many regulatory difficulties. The pooling of serum or plasma from different donors is regulatory limited to four donors due to the risk of transmission of viral infections. For the industrial processing of a larger pool of serum/plasma, the introduction of a virus removal procedure is mandatory. However, most virus inactivation methods are not suitable. For example virus inactivation via heat treatment or radiation leads to protein damage, virus inactivation via addition of detergents, for example TNBP-Tween 80 and/or Triton 100 is not allowed for the production of eye drops due to residues of these detergents, and virus filtration is not applicable since filtration of serum or plasma leads to clogging of the filter pores.

The present disclosure, not part of the claimed invention, provides a method for obtaining biologically active blood fractions after ultracentrifugation of blood serum or blood plasma that can be used for the treatment of damaged epithelial surfaces, in particular in connection with eye diseases and that are devoid of lipids and lipoproteins. Usually those lipids lead to an increase of viscosity and have a detrimental effect on the filtration. When separating those from the other serum/plasma fractions, virus filtration is made possible.

SORENSEN 0 ET AL: "The human antibacterial cathelicidin, hCAP-18, is bound to lipoproteins in plasma", JOURNAL OF BIOLOGICAL CHEMISTRY, AMERICAN SOCIETY FOR BIOCHEMISTRY AND MOLECULAR BIOLOGY, vol. 274, no. 32, 6 August 1999 (1999-08-06), pages 22445-22451, discloses a method for fractionating plasma by means of ultracentrifugation, this process results in fractionizing blood plasma via ultracentrifugation in a tube into a top fraction, one fraction and a bottom fraction,

BOZIC ET AL: "Viscosity of Plasma as a Key Factor in Assessment of Extracellular Vesicles by Light Scattering", CELLS, vol. 8, no. 9, 6 September 2019 (2019-09-06), page 1046, similarly discloses a method for fractionating plasma from healthy donors by means of ultracentrifugation, this process results in fractionizing blood plasma via ultracentrifugation in a tube into a top fraction, one or more middle fraction(s) and a bottom fraction.

Thus, the present invention provides for the first time a method for the production of serum and plasma products derived from a pool of donors, in particular eye drops, which are in compliance with regulatory requirements as regards virus inactivation, for use in the treatment of eye diseases associated with damaged epithelial surfaces.

Further embodiments of the present invention will be apparent from the description, the Figures and Examples that follow.

### BRIEF DESCRIPTION OF THE DRAWINGS

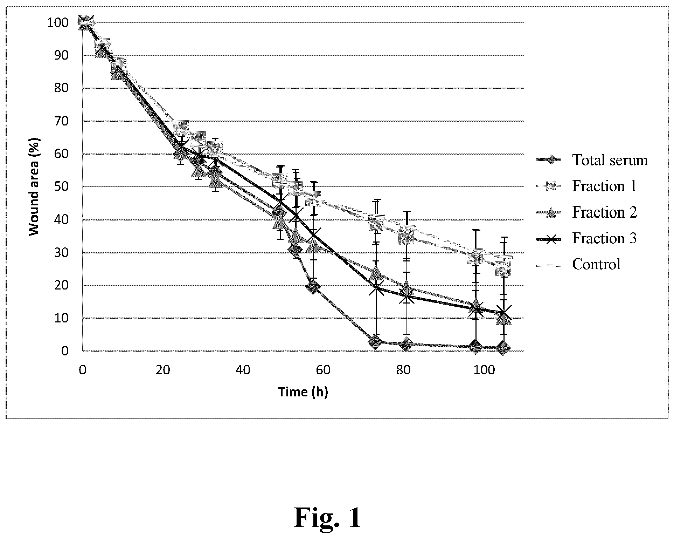

- **Fig. 1:**: Blood fractions 2 and 3, *i.e.* those which are substantially devoid of lipids, lipoproteins and extracellular vesicles show accelerated wound healing in an *in vitro* wound healing assay. The graph depicts the reduction of the wound area of human corneal epithelial cells (HCE-T cells) having a defined micro-injury (scratch) over a period 100 h after addition of serum fraction 1 (■), serum fraction 2 (▲) and serum fraction 3 (**×**) to the cell culture medium in comparison to the addition of total serum (◆) as well as to the control (-), *i.e.* KbM+.

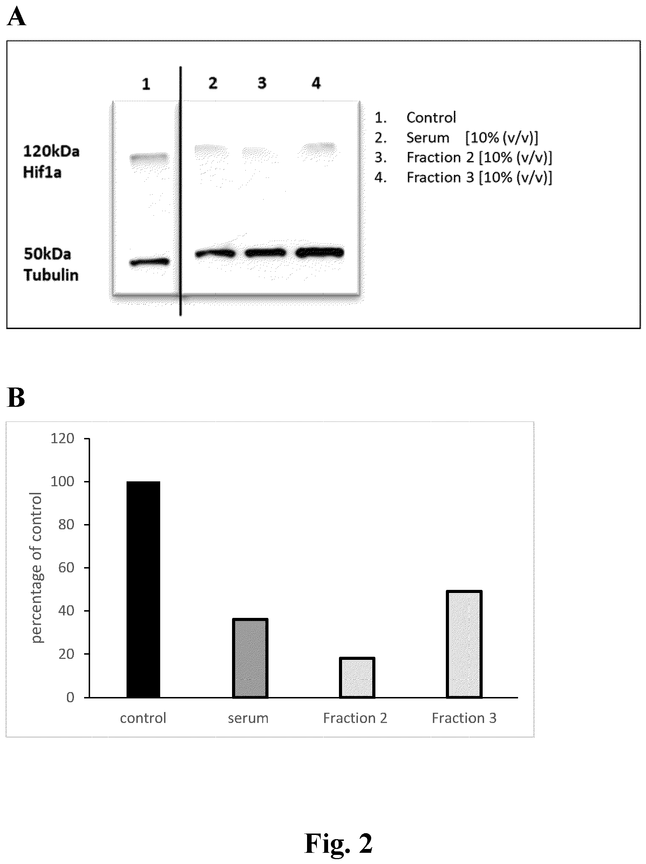

- **Fig. 2:**: Blood fractions 2 and 3 have a protective effect against cobalt chloride (CoCl₂) - induced oxidative stress in HCE-T cells as determined by HIF-1a protein level analysis by Western Blot. **Fig 2A****:** Western Blot showing the amount of HIF-1a protein compared to tubulin after growth of HCE-T cells in control medium KbM+ and after addition of serum, serum fraction 2 and serum fraction 3. **Fig. 2B****:** Bar chart showing the results of the semi-quantitative analysis by Western Blot described for Fig. 2A. In comparison to the control (set as 100%), much lower HIF-1a protein levels were observed in cells treated with either serum (36%), fraction 2 (18%), or fraction 3 (49%).

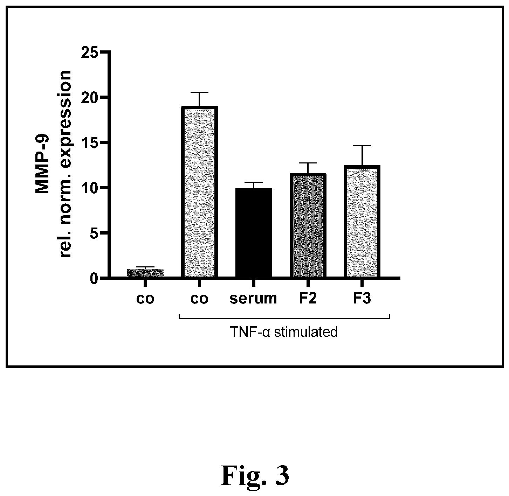

- **Fig. 3:**: Blood serum fractions 2 and 3 reduce tumor necrosis factor alpha (TNF-α)- induced inflammatory response in human corneal epithelial cells (HCE-T cells), exemplified by marker matrix metalloproteinase-9 (MMP-9). MMP-9 expression, determined by quantitative RT-PCR, was markedly increased in cells stimulated for 24 hours with TNF-α (10ng/ml) and this increase was reduced by treating the cells with 10% (v/v) of either total serum or the fractions F2 or F3, indicating anti-inflammatory properties of the serum fractions. (n=3 wells per treatment group, data are show as mean ±SD).

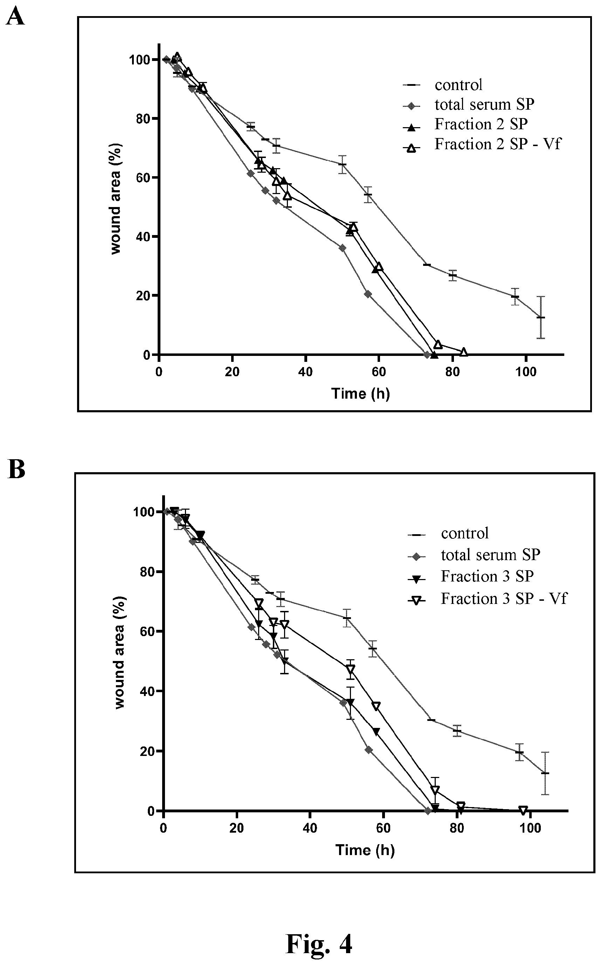

- **Fig. 4:**: Virus filtration (Vf) processed blood serum fractions 2 and 3 maintained their wound healing promoting effects in the *in vitro* wound healing assay with human corneal epithelial cells (HCE-T cells). The graph depicts the reduction of the wound area of HCE-T cells having a defined micro-injury (scratch) over a period 100 h after addition of the respective treatment to the cell culture medium. **Fig 4a****:** Accelerated wound healing, compared to the negative control (-), *i.e.* Keratinocyte growth medium (KGMoS) without supplements, was observed by treatment with total serum, serum pool (SP) (◆), by fraction 2 of SP (▲) and by the virus filtrated (Vf) fraction 2 of SP (Δ). **Fig 4b****:** Accelerated wound healing, compared to the negative control (-), *i.e.* Keratinocyte growth medium (KGMoS) without supplements, was observed by treatment with total serum (serum pool, SP) (◆), by fraction 3 of SP (▼) and comparable effects of the virus filtrated (Vf) fraction 3 of SP (V). (All serum-derived treatments were given at a volume equivalent to 500 µg total protein / ml medium (w/v); for all treatment groups n=3 wells per treatment group, data are show as mean ±SD).

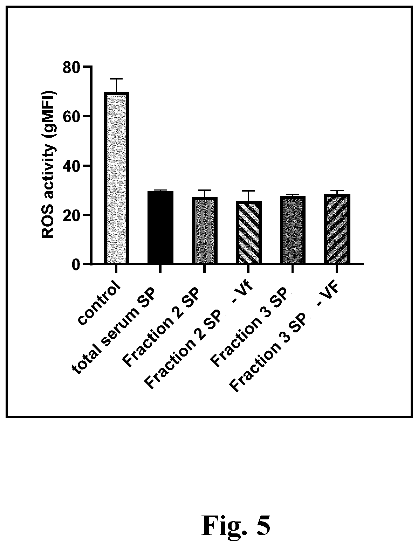

- **Fig. 5:**: Reactive oxygen species (ROS) activity of human corneal epithelial cells (HCE-T cells) stimulated with hydrogen peroxide (H₂O₂, 1.5 mM) is markedly reduced by blood serum fractions 2 and 3 as well as by respective virus filtration (Vf) processed blood serum fractions. ROS activity, plotted as geometric mean fluorescence intensity (gMFI), was determined by flow cytometry in H₂O₂, stimulated cells. In comparison to KGMoS-treatment (control) of stimulated cells, treatment with total serum (serum pool, SP) or with fraction 2 or fraction 3 of SP led to a reduction in stimulated ROS activity by around 50%. Similar reduction of ROS activity is observed by the virus filtrated (VF) fractions 2 or 3. All serum-derived treatments were given at a volume equivalent to 875 µg total protein / ml medium (w/v). (n=3 wells per treatment group, data are show as mean ±SD).

### DETAILED DESCRIPTION OF THE INVENTION

The present disclosure, not part of the claimed invention, relates to a method of obtaining biologically active blood fractions, in particular serum or plasma fractions that can be used for the treatment of damaged epithelial surfaces. In particular, the blood fractions are obtained via ultracentrifugation and subsequent separation from total serum or plasma and are preferably used for treating damaged epithelial surfaces in connection with eye diseases. '

When reference is made to (serum/plasma) fractions or one or more (serum/plasma) fraction(s), only fractions of blood serum and blood plasma, respectively, are meant and even when referring to "more fractions", the total serum and the total plasma, respectively is not encompassed.

As described in Example 2, human blood serum was subjected to ultracentrifugation at 100,000 × g for 24 hours and four visible fractions have been generated. The same fractionation will be achieved when using blood plasma (which also includes platelet-rich plasma) as starting material as also described in Example 2.

Ultracentrifugation is a common method for separating different fractions of a product according to their sedimentation properties and is known to the person skilled in the art. Conditions for obtaining the mentioned fractions of blood serum and plasma can be easily tested und visually verified. Thus, the method of the present invention comprises in a first step the fractionation of blood serum or blood plasma via ultracentrifugation in a tube into a top fraction, one or more middle fractions and a bottom fraction. In a preferred embodiment, the fractionizing results in two distinct middle fractions, which are surrounded by the top and the bottom fractions.

The top fraction (also designated as fraction 1 (F1)) as well as the two middle fractions (also designated as fraction 2 (F2) and fraction 3 (F3) have been analyzed regarding their biological activity. Already in 1984 Fox et al. (Arthritis Rheum 27 (1984), 459-461) reported the beneficial effects of autologous serum application to dry eye in Sjögren's syndrome. Later on, it was described that human serum eye drops not only function as a natural preservative-free lubricant, but also supply epitheliotrophic factors and essential substances for the recovery of a damaged epithelium; see for example Lekhanont et al., Sci Rep 6, (2016), 38143 as well as references cited therein. Autologous plasma eye drops have also been shown to be useful in treating dry eye; see Petznick et al., Eye 27 (2013), 1102-1114 and Alio et al., J Ophthalmol. (2017), 2457620. Thus, the beneficial effect of total serum and plasma on epithelial cells was already reported and total serum was used as a positive control in *in vitro* wound healing assays as described in Example 4.

As depicted in Fig. 1, serum fractions 2 and 3, *i.e.* the middle fractions, showed accelerated wound closure/wound healing in comparison to the control, *i.e.* KbM+ medium without serum. In particular, 60 to 70 hours after adding 1 % (v/v) of serum fraction 2 to wounded HCE-T cells, about 70 % of the wound area was closed and about 30 hours later, *i.e.* about 100 hours after adding 1 % (v/v) of serum fraction 2, about 90 % of the wound area was closed. The same result was observed after adding 1 % (v/v) of serum fraction 3 to the damaged HCE-T cells. In contrast, wound closure of about only 70% was reached in medium without addition of serum or serum fractions after about 100 hours. Serum fraction 1, *i.e.* the top fraction, showed a similar, even same behaviour than the control and did not show accelerated wound closure since only after about 100 hours, about 70 % of the wound was closed. In another experiment, wound closure/wound healing was even faster. As shown in Fig. 4, around 60 hours after adding serum fraction 2 or serum fraction 3 to wounded HCE-T cells, about 70 % of the wound area was closed and about 20 hours later, *i.e.* about 80 hours after adding serum fraction 2 or serum fraction 3, about 100 % of the wound area was closed. In contrast, wound closure of about only 80% was reached in medium without addition of serum or serum fractions after about 100 hours.

Thus, among the tested fractions, only fractions 2 and 3 are biologically active and are capable of inducing accelerated and improved wound healing in a HCE-T cell assay, wherein serum fraction 1 did not show such an activity. Since blood plasma after centrifugation similar like serum separates into a top fraction, middle fraction(s) and bottom fraction and since blood serum and blood plasma comprise substantially the same ingredients (blood serum may be defined as blood plasma without coagulation factors, in particular fibrinogens), alternatively plasma can be used as the starting material. Thus, the blood fraction(s) provided by the present invention can be obtained from both, serum and plasma and can be characterized by being capable of inducing wound healing in a HCE-T cell assay, preferably to the extend as mentioned above.

Biological activity of fractions 2 and 3 has also been shown in oxidative stress assays. In particular, it has been shown that both fractions have a protective effect against oxidative stress which is an important factor for determining their effectiveness in the treatment of eye diseases since oxidative stress, reflected by an imbalance between the intracellular generation of reactive oxygen species (ROS) and the antioxidant ROS scavenging system, is involved in various eye diseases as well as in ocular aging processes (for review see Cejka and Cejkova, Oxid. Med. Cell. Longev. 2015, doi: 10.1155/2015/591530, Dogru et al, Invest Ophthalmol Vis Sci 59 (2018) DES163-DES168).

For example, oxidative stress including hypoxia has been shown to disrupt the barrier function of epithelial cells as described in Cervelatti et al. Free Radical Research 48 (2014), 303-312 and in Terashini et al. Investigative Ophthalmology & Visual Science 49 (2008), 2432-2437 and contributes to various degenerative eye diseases including dry eye disease or Sicca syndrome; see Pflugfelder and Paiva, Ophthalmology 124 (2017), S4-S13. In particular, the anterior eye segment and mainly the corneal epithelium play an important role as they are directly exposed to a long list of environmental stresses which evoke permanently enhanced ROS generation and subsequently oxidative stress. Thus, detection of ROS generation is a measure for detecting oxidative stress. Furthermore, ROS and hypoxia-induced factors (HIF), *e.g.* HIF-1a, are early and partly interconnected factors regulated by oxidative stress and/or hypoxia. During oxidative stress/hypoxia, HIF-1a is stabilized and regulates various genes such as those involved in angiogenesis or immune response modulation. The stabilization of this protein is a hallmark of hypoxia, therefore detecting HIF is routinely used to screen for hypoxic stress response of cells.

As shown in Figures 2A and 2B, addition of 10 % (v/v) total serum, 10 % (v/v) serum fraction 2 and 10 % (v/v) serum fraction 3 reduced HIF-1a formation compared to the control, *i.e.* cells cultivated in KbM+. In particular, the HIF-1a protein level in cells treated with total serum was about 36 % in comparison to the control (100 %), in cells treated with serum fraction 2 about 18 % and in cells treated with serum fraction 3 about 49 %. Thus, fractions 2 and 3 have a protective effect against oxidative stress. Accordingly, the blood fraction(s) of the present invention have a protective effect against oxidative stress as determined by a CoCl₂ assay and lead to a reduced HIF-1a level as mentioned above. Furthermore, as shown in Fig. 5, serum fractions F2 and F3 are capable of reducing excessive ROS activation in human cornea epithelial cells. High levels of signal of intracellular ROS activity were observed after stimulation, but in cells treated with serum fractions F2 and F3, ROS activity was markedly reduced, proving that serum fractions F2 and F3 ameliorate oxidative stress by decreasing excessive ROS generation.

Moreover, inflammatory processes are recognized as underlying cause of various eye diseases, including dry eye diseases or Sicca syndrome (e.g., Baudouin et al, Acta Ophthalmologica 96 (2018), 111-119) or age-related macular degeneration (e.g., Kauppinen et al., Cell. Mol. Life Sci. 73 (2016), 1765-1786). Increasing evidence points towards a critical role of epithelial cells for both initiating and regulating ocular inflammatory processes. Increased levels of pro-inflammatory cytokines including tumor necrosis factor- alpha (TNF-α) (see review Roda et al, Int. J. Mol. Sci. 21 (2020), 3111) as well as increased levels of matrix metalloproteinases (MMP) like MMP-9 have been determined in tears of dry eye disease patients and MMP-9 is considered to represent a biomarker of inflammation as well as treatment efficacy in patients of dry eye disease (Baudouin et al, Acta Ophthalmologica 96 (2018), 111-119). *In vitro* studies have shown that stimulation of human corneal epithelial (HCE) cells by adding TNF-α to the culture medium triggers a pro-inflammatory response (Zheng et al., J. Cell Physiol. 222 (2010), 95-102) and results in an increase of MMP-9 (Li et al., Exp. Eye Res. 73 (2001), 449-459). In order to study the potential anti-inflammatory effects of serum fractions F2 and F3, an *in vitro* inflammation assay has been performed as described in Example 6. As shown in Fig. 3, stimulation of HCE-T cells by the pro-inflammatory cytokine TNF-α induces an increase in MMP-9 gene expression. Such pro-inflammatory response was markedly reduced by treating the TNF-α stimulated cells with either serum fraction F2 or F3 (10% v/v), an effect which was also observed in cells treated with total serum (10% v/v). Thus, the anti-inflammatory potency of total serum is maintained in serum fractions F2 and F3 of the present invention and those can be used for the treatment of various eye diseases in which inflammatory processes are recognized as underlying cause, including but not limited to dry eye diseases, Sicca syndrome, or age-related macular degeneration.

Accordingly, a further step of the method comprises the selection and isolation of either one of the middle fractions, *i.e.* fraction 2 or fraction 3, or both of the middle fractions that are biologically active and are capable of inducing wound healing in HCE-T cells as determined in an *in vitro* HCE-T cell assay.

Alternatively, or in addition the fraction or fractions are isolated and selected that have a protective effect against oxidative stress of HCE-T cells as determined in a CoCl₂ assay. Alternatively, or in addition the fraction or fractions are isolated and selected that reduce inflammatory response in HCE-T cells.

Alternatively, or in addition the fraction or fractions are isolated and selected that decrease excessive oxygen species (ROS) activity, *i.e.* counteract excessive ROS activity in HCE-T cells. Accordingly, with the help of the present invention, biologically active blood fractions have been isolated from total serum and total plasma, respectively, *i.e*. fractions 2 and 3 of the present invention. A further particular advantage of those fractions is that they are filterable through a virus filter in contrast to the total serum.

Plasma or blood serum of humans or animals has a potential risk of being contaminated with viruses. Therefore, the possibility of being infected with high risk viruses such as the human immunodeficiency virus and various hepatitis viruses cannot be denied, if a blood product manufactured using plasma or serum as a raw material is used. Thus, as mentioned above, validation of virus clearance is essential in the manufacture of blood products. In particular, products should be free of human immunodeficiency virus (HIV), Hepatitis B virus (HBV), Hepatitis C virus (HCV) and Hepatitis E virus (HEV). Preferably, products should be also free of Adenovirus (HAdV, especially type D), Herpes simplex virus (HSV), Epstein - Barr virus (EBV), Varicella-zoster virus (VZV), Cytomegalovirus (CMV), Parvovirus B19 and Hepatitis A virus (HAV).

Virus inactivation technologies are commonly used to fulfil viral safety. They include physical (*e.g.,* heat application, ultraviolet- and gamma irradiation) and chemical methods (*e.g*., solvent/detergent treatments like surfactants or methylene blue) or their combination (*e.g*., exposure to photosensitizer plus UV light). Common methods for virus capture also include filtration and chromatography in column or membrane configurations; see review of Junter and Lebrun, Rev Environ Sci Biotechnol 16 (2017), 455-489 as well as references cited therein.

However, physical or chemical virus inactivation methods have problems such as denaturation of proteins, requirements for complicated procedures for removing used chemical substances, and remaining of chemical substances in the finished products. A method of removing viruses using a membrane, on the other hand, is excellent as compared with the other methods, because this method does not cause proteins to become denatured, is free from a substantial decrease in the activity, and can increase safety of the products against viruses. Thus, in order to maintain biological activity of blood serum and plasma compounds, in particular of those compounds necessary for the treatment of the above-mentioned epithelial disorders, in particular those related to eye diseases, in particular to dry-eye diseases, and in order to ensure that there are no residual detergents in the pharmaceutical product, in particular in eye drops, viral filtration, also known as nanofiltration is the only option to produce a product ready for regulatory approval. However, nanofiltration is described to be applied only on plasma protein fractions and not on total plasma or serum since pores easily clog. For example, in Example 2 of US 7,592,134 B2 it is described that plasma filtration through a membrane having an average pore size of 75 nm was not successful since the pressure increased in a way that the experiment was discontinued.

Accordingly, currently there seems to be no option to generate pharmaceutical blood products, like serum/plasma based eye drops, which can be nanofiltered. Clogging of the filter pores might occur due to the presence of lipids and lipoproteins in the serum and plasma, respectively, which increase its viscosity.

Indeed, during ultracentrifugation of serum samples, hydrophobic lower density lipids (LDL) and lipoproteins accumulated in the upper part of the centrifugation tube and are thus comprised in the biologically inactive serum fraction 1. Discarding this fraction from the other fractions and selecting the fractions which are substantially free or free of those lipids and lipoproteins, respectively, decreased the viscosity and increased fluidity of the serum sample, *i.e.* the serum sample comprising the fraction or fractions of the present invention (fraction 2 and/or fraction 3). Accordingly, the method of the present invention comprises the selection of the two middle fractions which are free or substantially free of lipids, in particular LDLs and lipoproteins.

Thus, the present disclosure, not part of the claimed invention, relates to one or more blood fraction(s), in particular serum and plasma fractions, respectively which are characterized by being capable of inducing wound healing and/or having a protective effect against oxidative stress in a HCE-T cell assay, wherein said fraction(s) are free or substantially free of lipids and lipoproteins

Accordingly, the separation of blood, *i.e*. serum and plasma, respectively, into different fractions and subsequent isolation of the biologically active middle fraction(s), which are free or substantially free of lipids and lipoproteins while discarding fraction 1, which has been proven to be biologically inactive since it is not capable of accelerating wound healing in an HCE-T cell assay, leads to the possibility to perform a virus inactivation/removal step which does not affect the other compounds of the serum/plasma.

Thus, the present invention provides for the first time a possibility to produce blood based pharmaceutical products, in particular serum and plasma based eye drops including a virus removal/inactivation step via generating biologically active fractions comprising the advantageous components that are comprised in total serum and plasma, respectively, but without the filtration interfering lipids and lipoproteins as described above.

Most viruses range in size from approximately 20 to 200 nm, and the typical nanofilter has a pore size of 15 to 40 nm. Nanofiltration has proven effective in removing a wide range of viruses including the non-enveloped viruses and may even remove viruses smaller than the filter pore size. Proposed mechanisms for the removal of these small agents include viral adsorption at the charged membrane surface, aggregation of the smaller viruses, or association of the viruses with cellular components. Nanofiltration typically results in a 4- to 6-log removal of most viruses, with 90% to 95% recovery of protein activity; see Bryant and Klein, Arch Pathol Lab Med 131 (2007), 719-733. The terms virus reduction and virus inactivation are often used interchangeably. Both definitions are relative and neither guarantees sterility of a blood component. However, the filtration operation is usually classified as effective (log removal value, LRV > 4).

In principle all known and commercially available virus filter systems can be used in accordance with the present invention. Summaries of suitable virus filter systems are given for example in Junter and Lebrun, Rev Environ Sci Biotechnol 16 (2017), 455-489 and Burnouf and Radosevich, Haemophilia 9 (2003), 24-37. Virus filtration membranes are commonly made from poly(ethersulfone) (PES) (*e.g.,* Millipore Viresolve Pro; Virosart HS/HC/CPV from Sartorius Stedim Biotech, Aubagne, France, wherein the 20 nm filter retains ≥ 4 LRV of small non-enveloped viruses and ≥ 6 LRV of large enveloped viruses), poly(vinylidene fluoride) (PVDF) (*e.g*., Viresolve NFP Normal Flow Parvovirus from EMD Millipore; Ultipor VF Grade DV20/DV50 from Pall Corporation, Port Washington, NY; Planova^{™} BioEx, Asahi Kasei Medical, Tokyo, Japan ), and regenerated cellulose.

Among the latter, for example the commercial Planova^{™} filters (Asahi Kasei Medical, Tokyo, Japan) have been widely used to clear viruses from biologically produced pharmaceuticals, in particular blood products. They offer a choice of four nominal mean pore sizes, namely 15 nm (15N), 19 nm (20N), 35 nm (35N) and 72 nm (75N), wherein the large pore 75N model being essentially used as a pre-filter to remove impurities or aggregates prior to final virus filtration- and can be operated in normal (dead-end/flow through) or tangential flow (cross flow) filtration mode. Most efficient virus removal has been reported to be achieved with filters having a pore size of 15 nm to 35 nm. For example, Planova^{™} filters N15-N35, operated in the dead-end mode as a single filter or two units in series, were shown to provide LRV values ranging between 4 and 7. In the experiments of the present invention, pre-filtration has been performed with a Virosart Max filter (Sartorius, Göttingen, Germany; nominal pore size: 100 nm) connected to an Äkta Avant system to apply the required pressure of 0.2 MPa, wherein the pre-filtrate has been further filtered through a Virosart HC filter (Sartorius, Göttingen, Germany; nominal pore size: 20 nm).

Thus, in one embodiment the method of the present invention comprises a virus removal/inactivation/capture step, wherein the serum fractions and plasma fractions, respectively are subjected to filtration with a virus filter leading to fractions which are free or substantially free of viruses, for example free or substantially free of HIV, HAV, HBV, HCV, HEV, and Parvovirus B 19. In the context of the present invention, free or substantially free means that the virus load is reduced by 4 to 6 logs, *i.e.* the fractions have a LRV > 4, preferably > 5, more preferably > 6. In one embodiment, the LRV is between 4 and 8, between 4 and 7, or between 4 and 6. The term "virus filter" also includes folded membranes, which are preferably hydrophobic, or filter membranes and filters, respectively. The filters used in the method of the present invention have a pore size between 15 nm to 75 nm, preferably between 15 nm to 35 nm, more preferably between 15 nm to 20 nm, and most preferably of 20 nm. In a preferred embodiment, filter membranes are made from PES, PVDF, or regenerated cellulose, and most preferably from PES. In one embodiment, a single filter is used. In another embodiment, a series of filters is used, preferably two filters in series, wherein both filters preferably have a different pore size. For example, the fraction(s) are filtered through a first filter having an average pore size of 75 nm and subsequently through a second filter having an average pore size of 35 nm, or the fraction(s) are filtered through a first filter having an average pore size of 100 nm and subsequently through a second filter having an average pore size of 20 nm. Thus, in a preferred embodiment, a pre-filtration step is performed with a filter having a different pore size than the filter which is used for the subsequent/final filtration, preferably the pore size of the pre-filter is higher than the pore size of the filter used for subsequent/final filtration. More preferably, a pre-filter having a pore size between 75 nm and 300 nm, preferably between 75 nm and 100 nm, more preferably of 75 nm or 100 nm, most preferably of 100 nm is used. The pre-filter membrane can have a different material than the above-described filter membranes and can be for example polyamide. Furthermore, the pre-filter can be connected to a chromatography system, like an Äkta Avant system to apply a pressure of for example 0.2 MPa.

Thus, the fraction(s) of the present disclosure are substantially free or free of viruses.

Furthermore, it has been verified that virus filtration processed blood serum fractions 2 and 3 maintained their biological activity. For example, it has been shown that those fractions maintained their wound healing promoting effects in the *in vitro* wound healing assay with human corneal epithelial cells (HCE-T cells) and that those are also capable of reducing excessive ROS activation in HCE-T cells; see Examples 4 and 6 as well as Fig. 4 and Fig. 5. In particular, virus filtrated serum fractions 2 and 3 showed accelerated wound closure/wound healing in comparison to the control, *i.e.* Keratinocyte growth medium without supplements (KbM+), and similar wound closure/wound healing in comparison to non-virus filtrated serum fractions 2 and 3 as described above. Furthermore, high levels of signal of intracellular ROS activity were observed after stimulation but in cells treated with virus filtrated serum fractions F2 and F3, ROS activity was markedly reduced, proving that those fractions ameliorate oxidative stress by decreasing excessive ROS generation.

Viruses can be more efficiently removed when the permeability of useful components is ensured. Thus, the serum and plasma, respectively, used as a raw material for producing the blood fractions is preferably a fresh serum/plasma because serum/plasma stored for a long time before freezing causes a reduction in permeability and in the permeated amount during filtration operation due to proteins, lipids, and the like that have bonded during storage. Thus, serum/plasma with a short storage time before freezing is preferable.

As mentioned above, it is of particular interest to separate lipids and lipoproteins from the remaining serum and plasma, respectively. Ultracentrifugation at 100,000 × g for 16 to 24 hours allowed to separate the serum into the above-mentioned fractions allowing removal of the top fraction, *i.e.* non-active fraction 1 comprising most if not all of the hydrophobic lower density lipids (LDL) and lipoproteins from the starting sample which are strongly influential on the safe and reproducible virus-reducing filtration process. The content of lipids and lipoproteins is highly variable between blood donors and even within one donor as it strongly depends on factors like the meal of the previous day etc. Removal of that fraction allows to introduce virus-reduction by nanofiltration and leads to a standardisation of the production process for the serum-based and plasma-based eye drops.

In one embodiment, the method comprises an ultracentrifugation step at 100,000 × g to 120,000 × g for > 12 hours, preferably for 16 to 24 hours, more preferably at 100,000 × g for 24 hours. Since the different fractions are separated according to their sedimentation properties and once separated even a longer ultracentrifugation time will not change the order of the fractions. Of course, also a shorter centrifugation time or slower centrifugation is applicable, as long as the blood, *i.e.* serum/plasma is fractionated as mentioned above and a top fraction comprising the lipids and lipid proteins is formed. Various conditions can be easily tested and whether the fractions are formed can be verified visually. Whether the top fraction indeed contains lipids and lipoproteins and whether the other fractions, in particular the middle fractions, *i.e*. fractions 2 and 3 do not contain lipids and lipoproteins or at least to a lesser extent can be for example tested with the commercially available Sudan III reagent, which is a dye used for staining triglycerides and lipoproteins.

Since a majority of protocols applied for isolation of extracellular vesicles (EVs) and/or exosome are performed at 100,000 × g although usually for a shorter time, the bottom fraction in the tube obtained after running the method of the present invention comprises membrane-vesicles, *i.e.* EVs and exosomes. Detection of exosomes and EVs can be performed using various methods known to the person skilled in the art. Some of them, like electron microscopy or flow cytometry are summarized in Ko et al., Analyst. 141 (2016), 450-460. Thus, the fraction(s) of the present invention are substantially fee or free of exosomes or further membrane-vesicles. Since the top fraction comprises lipids and lipoprotein, and the bottom fraction comprises extracellular vesicles including exosomes, the one or more fractions in the middle, here two fractions, comprise the remaining serum/plasma components.

The method may either use serum and plasma, respectively that has already been prepared or may encompass a step of serum/plasma preparation from whole blood. The preparation of human serum and human plasma from full blood is a standard procedure and known to the person skilled in the art. In general, serum is collected after the blood is allowed to clot and plasma is collected after centrifugation of anti-coagulated blood as described in Example 1.

In a preferred embodiment, venous blood and serum/plasma from venous blood, respectively is used. In one embodiment, no umbilical cord blood and serum/plasma derived from umbilical cord blood, respectively is used. Thus, the fraction(s) of the present invention are derived from whole blood and preferably from venous blood, but not from umbilical cord blood.

As mentioned above, preferably blood from more than one donor is used since otherwise the amount of blood is limited and production of a blood-based product in industrial scale would not be possible and the whole production process not feasible. Thus, either the blood of different donors is pooled before serum/plasma preparation or the serum/plasma prepared from blood derived from different donors is pooled. In a preferred embodiment, the method of the present invention comprises the use of blood, serum and plasma, respectively derived from at least two, preferably more than four donors. Accordingly, the fraction(s) of the present invention are prepared from blood or serum and plasma, respectively derived from two and preferably from more than four donors. In one embodiment, the serum and plasma, respectively used in the present invention is allogenic, *i.e.* it is derived from a person other than the person to be treated with the serum/plasma product. In a further embodiment, the fractions are derived from a serum or plasma pool, which comprises allogenic serum/plasma. Since the composition of blood is variable between blood donors and depends on the state of health of the donor, it is further preferred to use blood/serum/plasma from healthy human male donors which have also been tested for virus contaminants as well as further pathogens as usually performed after blood donation. Preferably, before fractionizing, the serum and plasma, respectively is filter sterilized to remove pathogens, for example bacterial contaminations. In this context, any commercial filter applicable for sterile filtration may be used which pore size is usually in the µm range. Thus, in one embodiment a sterile filter is used having a pore size of 0.22 µm.

The present invention further relates to a liquid composition comprising either one of the middle fractions as obtained by the method of the present invention, *i.e*. fraction 2 or fraction 3, or which comprises both middle fractions, *i.e.* fractions 2 and 3. Thus, the composition of the present invention comprises one or two fractions as defined above and which have been obtained by the method as defined above. In one embodiment, the composition is completely composed of either one of the middle fractions, *i.e.* either fraction 2 or fraction 3 or of both of said fractions of the present invention without any further compounds, in particular without any further active compound or polymer.

In another embodiment, the composition further comprises a physiological aqueous solution, typically having a pH comprised between 7 and 8, preferably saline, more preferably normal saline. Preferably, the composition of the present inventing does not comprise any further compound as mentioned above except the fraction(s) and saline.

Saline is a mixture of sodium chloride in water and has a number of uses in medicine and is commonly used for the preparation of eye drops.

In one embodiment, the ratio of serum/plasma fraction to saline is 100% to 0%, in another embodiment it is 90% to 10%, in yet another embodiment it is 75% to 15%, in yet another embodiment it is 50% to 50%, in yet another embodiment it is 40% to 60%, in yet another embodiment it is 30% to 70%, in yet another embodiment it is 25% to 75%, in yet another embodiment it is 20% to 80%, in yet another embodiment it is 10% to 90%, in yet another embodiment it is 1% to 99%. Of course, the ratios in between are also encompassed by the present inventing in that the composition of the present invention comprises saline in a range of 0% to 99% and the fraction(s) in a range of 100% to 1%, preferably saline in a range of 50% to 80% and the fraction(s) in a range of 50% to 20%. As already mentioned above, either fractions 2 and 3 can be used separately or mixed together.

In one embodiment, the composition of the present invention and the fraction(s), respectively can comprise one or more pharmaceuticals, which for instance may further assist in the treatment of epithelial diseases, for example dry eye, such as cyclosporine. Other substances, excipients, etc., may be added to provide the composition with particular characteristics, *e.g*., for storage, delivery, application, such as hydrogel and combination with other substances (*e.g*. pharmaceuticals), etc. For instance, the viscosity of the composition can be adjusted by adding saline or further compounds, such as hyaluronic acid.

The blood, *i.e.* serum/plasma fractions, *i.e.* either fraction 2 or 3 alone or both fractions in combination and the composition, respectively of the present invention can be used for the treatment of various diseases associated with damages of the epithelial layers in the eye, in particular with non-keratinized epithelial cells. Various studies have already been performed as regards the use of autologous blood serum or plasma in the treatment of eye diseases, in particular of dry eye, for example as described in Fox et al. Arthritis Rheum 27 (1984), 459-461; Lekhanont et al., Sci Rep 6, (2016), 38143; Geerling et al., British Journal of Ophthalmology 88 (2004), 467-1474; Petznick et al., Eye 27 (2013), 1102-1114 and Alio et al., J Ophthalmol. (2017), 2457620. Thus, since the blood fraction(s) of the present invention have been shown to accelerate wound healing in HCE-T cells, they can be regarded as suitable for the treatment of various eye disease in a similar way than products comprising total serum or plasma. Furthermore, the depletion of fraction 1 does not affect the suitability of the fraction(s)/the composition since it is not biologically active. To the contrary, the depletion of fraction 1 is even favorable since otherwise virus filtration would not have been possible as mentioned above.

As mentioned above, serum fractions F2 and F3 show anti-inflammatory potency and thus, those can be used for the treatment of various eye diseases in which inflammatory processes are recognized as underlying cause, including but not limited to dry eye diseases, Sicca syndrome, or age-related macular degeneration.

Damage of the epithelial layer can occur through physical damage of the eye, but mainly due to an insufficient supply of tear fluid. In particular, while the corneal demand for glucose, electrolytes, and amino acids is supplied by the aqueous humour, growth factors, vitamins and neuropeptides, which are secreted by the lacrimal gland, support proliferation, migration, and differentiation of the ocular surface epithelia. As part of inflammatory processes additional proteins such as the adhesion factor fibronectin, complement factors, and antimicrobial proteins (for example, lactoferrin, immunoglobulins) are released into the tears from conjunctival vessels. Tears thus have lubricating, mechanical, but also epitheliotrophic and antimicrobial properties. A reduction of epitheliotrophic factors or their carrier compromises the integrity of the surface epithelia. This can lead to epithelial defects, which as a result of compromised wound healing persist and progress. In this situation it is important to lubricate the ocular surface - however, the ideal tear substitute should, in addition, provide epitheliotrophic support; see Geerling et al., British Journal of Ophthalmology 88 (2004), 467-1474. Such a support can be provided by the fraction(s) and compositions, respectively of the present invention.

The dry eye syndrome is a large and growing problem and is very common with an estimated 25% of patients in general ophthalmology or optometry clinics reporting symptoms of dry eye syndrome. It has multiple causes that result in an unstable tear film and rapid tear film breakup time. Dry eye syndrome is a multifactorial disease of the tears and ocular surface that results in symptoms of blurry vision and discomfort including foreign body sensation, irritation, burning, and light sensitivity. Dry eyes can have different causes, for example failure of the lacrimal glands to produce enough of the watery component of tears to maintain a healthy eye surface (aqueous tear-deficient dry eye), or inflammation of the meibomian glands which usually make the lipid or oily part of tears that slows evaporation and keeps the tears stable (evaporative dry eye). Dry eyes can also result from treating inflammation of the surface of the eye, the lacrimal gland, or the conjunctiva; from increase in the surface of the eye, as in thyroid disease when the eye protrudes forward; or from surgery when the eye lids are kept too long open. Dry eyes can also be a side effect of medications such as antihistamines, nasal decongestants, tranquilizers, certain blood pressure medicines, Parkinson's medications, birth control pills and anti-depressants. Furthermore, skin disease on or around the eyelids can result in dry eye as well as diseases of the glands in the eyelids, such as meibomian gland dysfunction. In addition, pregnancy; hormone replacement therapy in women; refractive surgery known as LASIK; chemical and thermal burns that scar the membrane lining the eyelids and covering the eye; allergies; infrequent blinking, associated with staring at computer or video screens; both excessive and insufficient dosages of vitamins; and loss of sensation in the cornea from long-term contact lens wear can cause dry eyes. Dry eye can be further associated with immune system disorders such as Sjogren's syndrome, lupus, and rheumatoid arthritis. Dry eye can be a symptom of chronic inflammation of the conjunctiva, the membrane lining the eyelid and covering the front part of the eye, or the lacrimal gland. Chronic conjunctivitis can be caused by certain eye diseases, infection, and exposure to irritants such as chemical fumes and tobacco smoke, or drafts from air conditioning or heating.

Thus, the composition of the present invention can be used for treating aqueous tear-deficient dry eye, which is a disorder in which the lacrimal glands fail to produce enough of the watery component of tears to maintain a healthy eye surface, for treating evaporative dry eye, which may result from inflammation of the meibomian glands, also located in the eyelids, or for treating dry eye associated with or resulting from treating inflammation of the surface of the eye, the lacrimal gland, or the conjunctiva. The composition and fraction(s), respectively of the present invention can be used for treating dry eye associated with any disease process that alters the components of the tears, for treating dry eye associated with an increase in the surface of the eye, as in thyroid disease when the eye protrudes forward, and/or for treating dry eye associated with a cosmetic surgery, for example, if the eyelids are opened too widely during surgery.

The composition of the present invention can be further used for ameliorating a symptom of dry eye syndrome, including: stinging or burning of the eye; a sandy or gritty feeling as if something is in the eye; episodes of excess tears following very dry eye periods; a stringy discharge from the eye; pain and redness of the eye; episodes of blurred vision; heavy eyelids; inability to cry when emotionally stressed; uncomfortable contact lenses; decreased tolerance of reading, working on the computer, or any activity that requires sustained visual attention; and/or eye fatigue.

The composition of the present invention can be further used for treating or ameliorating a symptom associated of dry eye syndrome in a subject exposed to a risk factor of temporary or chronic dry eye such as: side effect of some medications, including antihistamines, nasal decongestants, tranquilizers, certain blood pressure medicines, Parkinson's medications, birth control pills and anti-depressants; skin disease on or around the eyelids; diseases of the glands in the eyelids, such as meibomian gland dysfunction; pregnancy; hormone replacement therapy in women; refractive surgery known as LASIK; chemical and thermal burns that scar the membrane lining the eyelids and covering the eye; allergies; infrequent blinking, associated with staring at computer or video screens; both excessive and insufficient dosages of vitamins; homeopathic; loss of sensation in the cornea from long-term contact lens wear; immune system disorders such as Sjogren's syndrome, lupus, and rheumatoid arthritis (Sjogren's leads to inflammation and dryness of the mouth, eyes, and other mucous membranes); chronic inflammation of the conjunctiva, the membrane lining the eyelid and covering the front part of the eye, or the lacrimal; surface increase of the eye, as in thyroid disease when the eye protrudes forward; after cosmetic surgery if the eyelids are opened too widely or exposure keratitis, in which the eyelids do not close completely during sleep.

The composition of the present invention can be further used for treating dry eye syndrome in a subject in need of such treatment by increasing the tear film breakup time in the eyes of the subject. Break-up of the tear film is initiated at points where the surface of the corneal epithelium is irregular and where epithelial cells have been damaged or have lost their wettability. Active components in the serum fractions of the present invention can promote the health of the corneal epithelium, especially with improved stratification and differentiation of corneal epithelial cells that produce a surface glycocalyx.

The composition of the present invention can be further used for reducing, ameliorating, or preventing damage to the corneal epithelium, for example as indicated by the uptake of Rose Bengal dye.

In subjects suffering from diabetic retinopathy and macular degeneration it has been shown that the HIF-1a protein level is increased in comparison to subjects which do not suffer from the mentioned diseases. Thus, it is desirable to decrease the HIF-1a level in order to treat diabetic retinopathy and macular degeneration. As shown in the CoCl₂ assay described in Example 5, the addition of serum fractions 2 and 3 to HCE-T cells leads to such a decrease and thus, it is prudent to expect that the serum and plasma derived fraction(s) and compositions, respectively of the present invention can be used for treating/ameliorating diabetic retinopathy and macular degeneration as well as further diseases coming along with an increase in the HIF-1a protein level.

Non-keratinized epithelial surfaces are not only found in the eye but also throughout much of the body, except the limbs. Lubrication is continually bathing non-keratinized epithelial surfaces that can range from a thin watery aqueous solution to a thick, viscous fluid called mucus. Non-keratinized epithelial lubrication contains glycoproteins, proteoglycans, peptides, and enzymes that both promote and protect the health of epithelial cells. Not only does the lubrication protect the non-keratinized epithelial from mechanical irritation from frictional trauma, it also provides antimicrobial properties, preventing invasion from infectious organisms, a constant threat to these exposed areas. Non-keratinized epithelial surfaces form the lining of the cornea, conjunctiva, mouth, pharynx, esophagus, vocal cords, vagina, and cervix and play a vital role in the gastrointestinal, respiratory, and urogenital tracts.

These tissues are all sites of communication, where material and information are passed between the body and its environment. Because of their physiological functions of sensory activity (eyes, nose, mouth, and throat), gas exchange (lungs), food absorption (gut), and reproduction (uterus, vagina, and breast), the non-keratinized epithelial surfaces are by necessity dynamic, thin, permeable barriers to the interior of the body. These properties make these tissues particularly vulnerable to subversion and breach by pathogens.

Thus, it is prudent to expect that compositions/fraction(s) useful for the moisturizing and/or repairing of human corneal epithelial cells (HCE-T cells) as shown in Example 4 as well as the corresponding plasma products are also useful for the treatment of further diseases associated with damage of the non-keratinized epithelial cells such as dry mouth syndrome, vaginal dryness, diabetic ulcers and other chronic wounds.

Tissue damaging of the eye can also occur by the action of immune cells and the expression of pro-inflammatory cytokines and chemokines inducing local inflammatory responses. Such inflammatory and immune-mediated diseases which involve the anterior or posterior segment of the eye are for example Sjögren's syndrome (SS) dry eye, corneal allograft rejection, uveitis, and age-related macular degeneration (AMD). A summary of modern therapeutic approaches for non-infectious ocular diseases involving inflammation is given in Ratay et al., Advanced Healthcare Materials 6 (2017), doi: 10.1002/adhm.201700733.

Since the nerve growth factor-β (NGF-β), which is a powerful neurotrophic factor supporting the growth and survival of nerve cells and glia and which is in combination with the other factors present in serum and plasma, respectively responsible for neuroprotective and anti-inflammatory effects, is also present in the blood, *i.e.* serum and plasma fraction(s) of the present invention, it is prudent to expect that the fraction(s) and composition of the present invention are also suitable for the treatment of neurodegenerative ophthalmological pathologies as described in WO 2018/229718.

Thus, the composition of the present invention is used in treating, preventing and/or ameliorating ophthalmic disorders coming along with epithelial damage and/or eye diseases in which inflammatory processes and oxidative stress are recognized as underlying cause, in particular in treating, preventing and/or ameliorating the diseases and/or conditions selected from - but not limited to - dry eye disease or effects of dry eye, further wetting disorders of the cornea, ocular surface diseases, physical damage to the surface of the eye, eye fatigue, Sicca syndrome, Sjögren's syndrome, age-related macular degeneration, diabetic retinopathy, corneal allograft rejection, dry eye in HIV-patients, and/or autoimmune uveitis.

Thus, the composition of the present invention is preferably designed to be administered topically to or adjacent to the diseased surface, in particular the disordered epithelial surface. In a preferred embodiment, the composition of the present invention is administrable to the surface of the eye.

In one embodiment, the composition is formulated in liquid form, for example as drops, in gel form or as ointment, crème, or spray. Preferably, the composition is formulated as eye drops. In another embodiment, the composition of the present invention is designed to be administered by injection into body tissues adjacent damaged or disordered epithelium, preferably intravitreal. Injectable compositions may be required when epithelial damage goes deep.

In one embodiment, the composition of the present invention is provided in form of eye drops. Thus, the present invention relates to eye drops comprising or consisting of the composition of the present invention. Preferably, the eye drops do not comprise any further ingredients except the fraction(s) of the present invention, or except said fraction(s) and saline in the concentrations mentioned above in connection with the composition of the present invention.

In another embodiment, the full range of lubricants/viscosity modifiers used in conventional artificial tears may be used for the preparation of the eye drops of the present invention, such as hypromellose (hydroxypropyl methylcellulose), carbomer gel (high molecular weight polyacrylates, usually cross linked with a polyol), carmellose (carboxymethyl cellulose) and sodium hyaluronate (a natural or synthetic high molecular weight glycosaminoglycan, which is naturally present in epithelial cells, and so is particularly suitable for use in epithelial treatments). These "artificial tears" of conventional form are particularly suitable for mixing with the fraction(s) to form the eye drops of the present invention, since they lubricate the eye and have similar rheology to natural tears, ensuring that they remain spread across the surface of the eye for long enough for the healing components of the blood to act on the epithelial cells. Indeed, it may be beneficial to arrange for the artificial tears to have a higher viscosity than natural tears, to maximize residence time. It is known to formulate artificial tears with high levels of sodium hyaluronate such that they gel slightly on administration to the eye, further increasing the residence time of the active components of the eye drops (*i.e.* in the present case, the active components of the blood serum/plasma fractions).

The present invention also relates to the corresponding treatment methods for treating, ameliorating, or preventing the above-mentioned diseases which comprises administering the fraction(s), preferably in form of the compositions of the present invention or in form of the eye drops of the present invention adjacent to or to the epithelial surface to be treated in a therapeutically effective dose. Administration can be performed either topically, as injection or intravitreal in the forms as explained above. A therapeutically effective dose can be easily tested by a person skilled in the art and depends on the subject and disease to be treated. For example, in case of ameliorating a symptom of dry eye syndrome, for example stinging or burning of the eye or a sandy or gritty feeling as if something is in the eye, the eye drops/composition of the present invention can be administered as soon as the ameliorating effect ceases. Furthermore, the fraction(s) and composition of the present invention can be used for cosmetic purposes since the active ingredients in the serum can help in regenerating cells.

The eye drops of the present invention, the composition of the present invention can be provided in any suitable container. Thus, the present invention relates to single doses or multiple doses container comprising the eye drops, the composition, of the present invention, preferably wherein the container is an ampule, preferably comprising a pump system. Associated with such container can be a notice in the form prescribed by a governmental agency regulating the manufacture, use or sale of pharmaceuticals or biological products, which notice reflects approval by the agency of manufacture, use or sale for human administration.

The present invention further related to a method of manufacture of eye drops. The method comprises fractionizing total serum and plasma, respectively, via ultracentrifugation as described above. The serum/plasma is prepared from whole blood, preferably from venous blood and not from umbilical blood as explained above. Before use, the blood is tested for pathogens, *i.e.* bacterial and virus contaminations and the serum/plasma is filter sterilized in order to remove bacteria, e.g. with a 0.22 µm filter as described above. In a preferred embodiment, the serum/plasma is derived from four donors. In a next step, either one of the biologically active middle fractions, *i.e.* fraction 2 or fractions 3 or both of the middle fractions obtained after ultracentrifugation are isolated. As outlined in detail further above, the isolated fractions are characterized by being capable of inducing wound healing and/or having a protective effect against oxidative stress and/or reducing inflammatory response and/or decreasing excessive reactive oxygen species (ROS) activity in a HCE-T cell assay, and which are free or substantially free of lipids and lipoproteins and preferably also free or substantially free of exosomes. In an optional step, saline is added to the fraction(s). Thus after isolation of the fraction(s) (and optionally dilution with saline to the above-indicated concentrations), nanofiltration is followed in order to reduce the amount of viruses. This procedure, as well as reference to suitable filters is explained in detail further above. However, preferably, a filter with a pore size between 35 nm and 15 nm is used. Afterwards, the nanofiltered fraction(s) which are optionally mixed with saline are filled into suitable containers. Those are mentioned above. In order to keep the eye drops fresh, they might be stored at -20°C but can also be used directly.

Several documents are cited throughout the text of this specification. However, there is no admission that any document cited is indeed prior art as to the present invention.

A more complete understanding can be obtained by reference to the following specific Examples which are provided herein for purposes of illustration only and are not intended to limit the scope of the invention.

### EXAMPLES

### Example 1: Human serum and plasma preparation

The preparation of human serum and plasma from full blood is a standard procedure and known to the person skilled in the art.

In general, serum is collected after the blood is allowed to clot. The clot is removed by centrifugation and the resulting supernatant, designated serum, is carefully removed and collected. For example, after collection of the whole blood, the blood is allowed to clot by leaving it undisturbed at room temperature for about 1 hour. Afterwards, the clot was removed by centrifugation at 3,000 × g for 10 to 15 minutes.

Alternatively, plasma of human full blood can be prepared, for example via collecting whole blood into anticoagulant-treated tubes e.g., EDTA-treated or citrate-treated or heparinized tubes. Afterwards, cells are removed from plasma by centrifugation, for example for 10 minutes at 1,000 - 2,000 × g using a refrigerated centrifuge. The resulting supernatant is designated plasma. Standard operating procedures for serum and plasma collection are described in Tuck et al., J Proteome Res. 8 (2009), 113-117.

Sterile filtration was performed using a 0.22 µM polyethersulfon syringe filter (Sartorius AG, Göttingen, Germany) and the serum/plasma is either directly used or frozen at -20°C until further processing.

### Example 2: Human blood fractionation

Centrifugation of the human serum obtained as described in Example 1 was performed at 3,000 × g for 10 min. Afterwards, the serum was fractionated at 100,000 × g for 24 h. In brief, 15 mL of serum were transferred into Beckman centrifuge tubes (364664), combined with a floating spacer (355536) and centrifuged overnight in a Js 24-38 Rotor at 100,000 × g by usage of an Avanti J-30 ultracentrifuge. Four distinct fractions have been formed, *i.e.* a top fraction (F1), a bottom fraction (F4) and the two fractions in the middle (F2 and F3). The visible separated fractions F2 and F3 were extracted and stored at -80°C until further usage. A similar approach is used for the fractionation of plasma obtained as described in Example 1.

### Example 3: Virus filtration of serum fraction 2 (F2) and serum fraction 3 (F3)

To remove residual viral contaminations, human serum fractions F2 and F3 obtained from ultracentrifugation of human blood serum (serum pool (SP) of healthy male donors, n=5) were filtered through a combination of filters, according to the manufacturer's user manual. In a first step, 15 ml of the respective serum fraction were diluted with sterile saline (NaCl 0.9%, B. Braun, Melsungen, Germany) to a protein concentration of 15 mg/ml (BCA protein assay kit, Thermo Scientific) and pre-filtered through a Virosart Max filter (Sartorius, Göttingen, Germany; nominal pore size: 100 nm) connected to an Äkta Avant system to apply the required pressure of 0.2 MPa. In a second step, 5 ml of the respective pre-filtrate were diluted with sterile saline to a protein concentration of 5 mg/ml and filtered through a Virosart HC filter (Sartorius, Göttingen, Germany; nominal pore size: 20 nm). The final protein concentration of all fractions was determined (BCA protein assay kit, Thermo Scientific) and all samples, SP, F2 and the virus-filtrated F2-Vf, F3 and the virus-filtrated F3-Vf, were stored at -80°C until further usage.

### Example 4: Human blood fractions induce wound healing in HCE-T cells

The fractions 1, 2 and 3 obtained by ultracentrifugation as described in Example 2 were examined for their biological activity in an *in vitro* wound healing assay. In particular, the wound healing assay on human corneal epithelial cells (HCE-T cells) was performed to investigate the proliferative and migration-promoting properties of serum and serum fractions.

The assay is based on *an in vitro* cell culture test in which a defined micro-injury (scratch) is added to a monolayer of HCE-T cells and the subsequent time-dependent wound closure is recorded microscopically via image analysis software depending on the application of serum, serum fractions and other samples. The assay thus allows the required precise analysis of parameters influencing cell migration and proliferation.

The assay system used is based on well-described methods and has already been frequently used as a model for corneal wound healing; see Maugeri et al., Peptides 99 (2018), 20-26; Schicht et al., Scientific Reports 8 (2018), 9791; Gonzalez-Andrades et al., Sci Rep 6 (2016), 2849; Liang et al., Molecular vision 18 (2012), 2195-2204; and Zelenka and Smith, Expert Opinion on Therapeutic Patents 15 (2005), 875-887 and has also been used to investigate serum effects; see Robciuc et al., Eye (London, England) 32 (2018), 813-819; and Wu et al., International journal of ophthalmology 10 (2017), 908-913.

HCE-T cells (Araki-Sasaki et al., Investigative ophthalmology & visual science 36 (1995), 614-621) were obtained from the RIKEN cell bank (Tsukuba, Japan) and cultivated in serum-free Keratinocyte growth medium (KGM), consisting of keratinocyte basal medium (KbM) supplemented with specific single quotes of growth factors and antibiotics (KbM and single quotes have been obtained from Lonza, Ch-Basel). Additionally, calcium chloride solution (0.25 M, Acros Organics, Thermo Fisher Scientific, USA) was added to the KGM to reach a final concentration of 0.5 mM calcium chloride in the KGM.

For the wound healing assay, HCE-T cells were seeded in 24-well plates (TPP, Ch-Trasadingen) (90,000 cells per well) and cultivated in KGM medium for another seven days, resulting in a confluent cell layer. A sterile 1000 µl Eppendorf pipette tip (Eppendorf, D-Wesseling-Berzdorf) was used to create the wound (scratch) in the cell monolayer. The wells were then rinsed twice with PBS (MP Biomedicals, D-Eschwege) to remove the detached cells. For the time of the wound healing experiment, cells were kept either on KbM+ (KbM containing antibiotics and 0.5 mM Calcium chloride) without serum or serum fractions or KbM + with 1% (v/v) of either serum or the respective serum fraction (F1, F2, F3). After addition of the samples, the cells were analysed under the Olympus microscope type IX50 (Olympus, D-Hamburg) at fixed time points until wound closure, but for a maximum of 5 days. Six photos per well were taken at each measurement point. The wound area was determined with the ImageJ-win64 software and then evaluated with the Origin software by plotting the percentage wound area relative to the initial wound area against time.

As depicted in Fig. 1, serum fractions 2 and 3 shown accelerated wound closure/wound healing in comparison to the control, *i.e.* KbM+ medium without serum. In contrast, serum fraction 1 did not show accelerated wound closure/wound healing in comparison to the same control. Thus, in contrast to fraction 1, fractions 2 and 3 are biologically active.

The above described *in vitro* wound healing assay was also performed with human blood serum (serum pool (SP) of healthy male donors, n=5) and virus filtrated serum fractions, wherein virus filtration was performed as described in Example 3. In particular, the serum pool (SP), serum fraction 2 of SP, virus filtrated serum fraction 2 of SP, serum fraction 3 of SP, and virus filtrated serum fraction 3 of SP were analysed. As depicted in Fig. 4, virus filtration processed blood serum fractions 2 and 3 maintained their wound healing promoting effects in the *in vitro* wound healing assay with human corneal epithelial cells (HCE-T cells).

### Example 5: Human blood fractions have a protective effect against oxidative stress in HCE-T cells

The corneal epithelium of the human eye has many important functions that includes the provision of a barrier that protects from harmful external agents, e.g. microbes and chemicals, allows the establishment of a distinct internal environment and maintains the corneal transparency. Oxidative stress including hypoxia have been shown to disrupt the barrier function of epithelial cells as described for example in Cervelatti et al., Free Radical Research 48 (2014), 303-312 and in Terashini et al., Investigative Ophthalmology & Visual Science 49 (2008), 2432-2437) and contribute to various degenerative eye diseases including dry eye disease or Sicca syndrome; see Pflugfelder and Paiva, Ophthalmology 124 (2017), S4-S13. Reactive oxygen species (ROS) and hypoxia-induced factors (HIF), e.g. HIF-1a, are early and partly interconnected factors regulated by oxidative stress and/or hypoxia. During oxidative stress/hypoxia, HIF-1a is stabilized and regulates various genes such as those involved in angiogenesis or immune response modulation. The stabilization of this protein is a hallmark of hypoxia, therefore detecting HIF is routinely used to screen for hypoxic stress response of cells.

In order to analyse a potentially protective effect of serum and the serum fractions on oxidative stress / hypoxia in HCE-T cells, a cobalt chloride (CoCl₂) assay was performed. CoCl₂ is commonly used as oxidative stressor and the induction of cellular hypoxia by activating and stabilizing HIF-1a through incubation with CoCl₂ is well established; see Cervelatti et al., Free Radical Research 48 (2014), 303-312.

HCE-T cells were seeded at 25,000 cells/well in a 24-well plate and grown over night under standard conditions in serum-free Keratinocyte growth medium (KGM, as described in Example 4). On the next day, cells were treated with CoCl₂ to induce hypoxia/oxidative stress according the protocol of Wu and Yotna, Journal of Visualized Experiments 54 (2011), e289). In brief, cells were incubated with 100 µM CoCl₂ added to KbM+ under standard conditions (37°C, 5% CO₂) for 24 h. Additionally, growth factors were replaced with 10 % (v/v) of either total serum or the respective human serum fractions, fractions 2 or 3, obtained after ultracentrifugation as described in Example 2.

HIF-1a expression was analyzed via Western Blot. In particular, after overnight CoCl₂ treatment, cells were lysed by addition of 100 µl of PBS, containing 5% SDS. The cell debris was removed by centrifugation. After addition of loading buffer and heating at 95°C for 5 min, 20 µl of the samples were loaded on a 10 % SDS-polyacrylamide gel. After electrophoresis and transfer to nitrocellulose membrane, the latter was blocked with 5% non-fat milk in TBS-T buffer for 2h at 4°C. Immunoblot was performed over night with mouse anti-human HIF-1-alpha primary monoclonal antibody (BD Biosciences) (1:1000) in TBS-T at 4°C. Alpha tubulin served as loading control (Santa Cruz). On the next day, the membrane was washed three times with TBS-T for 5 min at RT and incubated for 1h with HRP-conjugated secondary antibody (1/10000 in TBS-T). After three final washing steps with TBS-T, protein bands were visualized by addition of HRP-substrate (Luminata Forte, Merck) and chemiluminescence was detected by an ECL machine (Chemocam Imager, INTAS).

In a first experiment, the activation of the formation of the protein HIF-1a by the addition of CoCl₂ (0 - 300 µM) was tested. Therefore, total protein was isolated from each batch to investigate the expression of HIF-1a at the protein level by Western blotting in comparison to CoCl₂ untreated control cells. A concentration-dependent CoCl₂ induced increase in HIF-1a has been observed in HCE-T cells incubated in keratinocyte basal medium (KbM+, KbM containing antibiotics and 0.5 mM Calcium chloride); data not shown. For further experiments, CoCl₂ at a concentration of 100 µM was used for HIF-1a activation.

Based on these first experiments, HIF-1a activation was determined depending on the addition of serum and serum fractions 2 and 3. It was shown that the addition of serum and the fractions F2 and F3 obtained as described in Example 2 reduced HIF-1-alpha formation compared to the control as shown in Fig. 2A and 2B. In particular, a semi-quantitative analysis, calculating the relative levels of grey value for each HIF-1a band (by taking the respective loading control into consideration) and comparing it to control (set as 100%), revealed much lower HIF-1a protein levels in cells treated with either serum (36%), Fraction 2 (18%), or Fraction 3 (49%); see Fig. 2B.

### Example 6: Serum Fractions reduce inflammatory response

Inflammatory processes are recognized as underlying cause of various eye diseases, including dry eye diseases or Sicca syndrome (e.g., Baudouin et al, Acta Ophthalmologica 96 (2018), 111-119) or age-related macular degeneration (e.g., Kauppinen et al., Cell. Mol. Life Sci. 73 (2016), 1765-1786). Increasing evidence points towards a critical role of epithelial cells for both initiating and regulating ocular inflammatory processes. Increased levels of pro-inflammatory cytokines including tumor necrosis factor- alpha (TNF-α) (see review Roda et al, Int. J. Mol. Sci. 21 (2020), 3111) as well as increased levels of matrix metalloproteinases (MMP) like MMP-9 have been determined in tears of dry eye disease patients and MMP-9 is considered to represent a biomarker of inflammation as well as treatment efficacy in patients of dry eye disease (Baudouin et al, Acta Ophthalmologica 96 (2018), 111-119). *In vitro* studies have shown that stimulation of human corneal epithelial (HCE) cells by adding TNF-α to the culture medium triggers a pro-inflammatory response (Zheng et al., J. Cell Physiol. 222 (2010), 95-102) and results in an increase of MMP-9 (Li et al., Exp. Eye Res. 73 (2001), 449-459).

In order to study the potential anti-inflammatory effects of serum fractions 2 and 3, an *in vitro* inflammation assay in human corneal epithelial cells (HCE-T cells) was performed. The pro-inflammatory cytokine TNF-α was used as inductor and MMP-9 expression in HCE-T cells was determined to indicate the level of inflammatory response of the cells.

For the inflammation assay, HCE-T cells were seeded in 6-well plates (TPP, Ch-Trasadingen) (250,000 cells per well) and cultivated in serum-free keratinocyte growth medium (KGM, as described in Example 4) for ten days under standard conditions (37° C, 5% CO₂). On the experimental day, culture medium was changed to KbM+ (as described in Example 4), and TNF-α (10 g/ml) was added to the medium. The respective treatment, *i.e.* control (KbM+ medium) or 10% (v/v) of either total serum or the respective human serum fractions 2 or 3, obtained after ultracentrifugation as described in Example 2, was added and the cells were incubated for 24 h under standard conditions.

MMP-9 expression was analyzed by real-time polymerase chain reaction, RT-PCR. After 24h of TNF-α stimulation and treatment, cells were trypsinized, centrifuged and the cell pellet was dissolved in 500µl TRIzol reagent (Life technologies, USA-Waltham). RNA was isolated by adding chloroform (Thermo Fisher scientific, USA-Waltham) followed by centrifugation for 15 min (12,000xg, 4°C, Beckman-Coulter), transferring the transparent upper phase into a new tube, incubate for 10 min at RT and centrifuge again for 10 min (12,000xg, 4°C, Beckman-Coulter). The RNA pellet was then washed two times with 75% ethanol (Thermo Fisher scientific, USA-Waltham), centrifuged for 5 min (7,500xg, 4°C), the supernatant discarded and the pellet dried briefly at RT before resuspension with 30µl DEPC-Water and dissolution by keeping it for 10 min at 57°C. RNA concentration of each sample was determined by means of a nanoquant-plate and a Tecan plate reader (Tecan, Ch-Männedorf). Of each sample, 1 µg of isolated RNA was used for cDNA synthesis by means of the iScript gDNA Clear cDNA synthesis kit (BioRad, D-Feldkirchen) according to the manufacturer's protocol. The resulting cDNA samples were frozen at -20°C until RT-PCR was performed. For RT-PCR of MMP-9 and the house keeping genes (GAPDH, β-actin) the CFX Connect RT-PCR detection system (BioRad, USA-California) with the following primer were used: MMP-9, forward: AGT ACC GAAG AGA AAG CCT ACT T, reverse: TGC AGG ATG TCA AAG CTC AC; GAPDH, forward: GAA GGT GAA GGT CGG AGT, reverse: GAA GAT GGT GAT GGG ATT TC; β-actin, forward: GCT ATT TGG CDC TGG ACT T, reverse: GCG GCT CGT AGC TCT TCT C. Briefly, a mastermix per primer pair was prepared, containing the iTaq Universal SYBRgreen supermix (BioRad, USA-California), the respective forward and reverse primer (Invitrogen, Thermo Fisher, USA-Waltham) and DEPC-water. Following the PCR according to the manufacturers protocol (program CFX-Maestro, BioRad, USA-California), melting curve analysis was done to check for unspecific products. For analysis of the results, the CFX-Maestro Software was used. Three biological replicates and for each of those, three technical replicates were investigated and the normalized gene expression of MMP-9 calculated.

As shown in Fig. 3, stimulation of HCE-T cells by the pro-inflammatory cytokine TNF-α induces an increase in MMP-9 gene expression. Such pro-inflammatory response was markedly reduced by treating the TNF-α stimulated cells with either serum fraction F2 or F3 (10% v/v), an effect which was also observed in cells treated with total serum (10% v/v). Thus, the anti-inflammatory potency of total serum is maintained in fraction F2 and F3, obtained by ultracentrifugation as described in Example 2.

### Example 7: Human blood fractions counteract excessive ROS activity in HCE-T cells

In various eye diseases as well as in ocular aging processes, oxidative stress reflected by an imbalance between the intracellular generation of reactive oxygen species (ROS) and the antioxidant ROS scavenging system, is involved (for review see Cejka and Cejkova, Oxid. Med. Cell. Longev. 2015, doi: 10.1155/2015/591530, Dogru et al, Invest Ophthalmol Vis Sci 59 (2018) DES 163-DES168). The anterior eye segment and mainly the corneal epithelium play an important role as they are directly exposed to a long list of environmental noxea which evoke permanently enhanced ROS generation and subsequently oxidative stress. The ability of the serum fractions F2 and F3 to ameliorate oxidative stress by decreasing excessive ROS generation was investigated *in vitro* by applying H₂O₂, a commonly used reagent to stimulate ROS activity (e. g. Ho J.H-C. et al, Br. J. Opthalmol. 92 (2008), 992-997) to cornea epithelial cells and measure treatment-induced changes of intracellular ROS signal by flow cytometry.

Human cornea epithelial cells, HCE-T cells (Araki-Sasaki *et al*, 1995) were seeded at 2 × 10⁵ cells/well in a 24-well plate in KbM+ medium (described in Example 4), kept under standard conditions (37°C, 5% CO₂) and left undisturbed for 1 hour. Thereafter, cells were stimulated by adding 1.5 mM H₂O₂ (Sigma Aldrich, Merck D-Darmstadt) and following 30 minutes, total serum (volume equivalent to 875 µg total protein) or the respective serum fractions (for each fraction, a volume equivalent to 875 µg total protein) were added to the wells. Cells were left undisturbed for another 3.5 hours and kept under standard conditions. For staining of ROS activity in the cells and measurement by flow cytometry, the CellROX green flow cytometry assay kit (Life technologies, Thermo Fisher Scientific) was used. Briefly, 2µl (0.7µM) of the cell-permeable CellROX green detection reagent (diluted 1:10 in DMSO) was added to the wells and incubated for 45 minutes in the dark at 37°C. Thereafter, 1 ml of PBS was added and the cells were transferred from the well into a tube and 200 µl of trypsin was added and incubated for 3 minutes before adding 1 ml PBS and a centrifugation step (300xg for 5 minutes at RT). The obtained cell pellet was washed with 5 ml cold buffer-CI (cell isolation buffer, IBA, D-Göttingen) and finally, cells were resuspended in 200 µl of cold buffer-CI and analysed in the flow cytometer. FlowJo software was used for analysing the data and the ROS signal was expressed as geometric mean fluorescence intensity (gMFI).

The results, as shown in Fig. 5, indicate that serum fractions F2 and F3 are indeed capable of reducing excessive ROS activation in human cornea epithelial cells. The signal of intracellular ROS activity is detectable only when the specific dye is oxidized by ROS formed in the cells. High levels of signal were observed after stimulation but in cells treated with serum fractions F2 and F3, ROS activity was markedly reduced, proving that serum fractions F2 and F3 ameliorate oxidative stress by decreasing excessive ROS generation.

In summary, both analyzed blood fractions are capable of inducing wound healing and have a protective effect against oxidative stress or pro-inflammatory stimulation in HCE-T cells. Furthermore, fractions 2 and 3 counteract the excessive ROS formation, thereby preventing the ocular surface from oxidative stress.

## Claims

1. A composition comprising one or more blood fraction(s), wherein said fraction(s) are free or substantially free of lipids and lipoproteins and obtained by a method comprising
(i) fractionizing blood serum or plasma via ultracentrifugation in a tube into a top fraction, one or more middle fraction(s) and a bottom fraction; and
(ii) selecting one or more middle fraction(s), wherein the selected fraction(s) are **characterized by** being capable of inducing wound healing and/or having a protective effect against oxidative stress and/or reducing inflammatory response and/or decreasing excessive reactive oxygen species (ROS) activity in a human corneal epithelial cells-transformed (HCE-T) assay,
wherein the composition is designed to be administered topically and preferably formulated as drops, ointment or gel.

2. The composition of claim 1, which further comprises a pharmaceutically acceptable carrier, preferably wherein the carrier does not induce irritation to the eye and is preferably saline.

3. The composition according to claim 1 or 2, wherein the selected fraction(s) are two middle fractions.

4. The composition according to any one of claims 1 to 3, wherein the selected fraction(s) at concentration of 1% (v/v) induce wound closure of about 70% of the wounded area within about 60-70 hours.

5. The composition according to any one of claims 1 to 4, wherein the selected fraction(s) are subjected to filtration with a filter having a pore size between 15 nm to 80 nm, preferably between 15 nm to 40 nm, more preferably between 15 nm to 35 nm, and even more preferably between 15 nm to 20 nm, and most preferably 20 nm, preferably wherein a pre-filtration step is performed with a filter having a different pore size, preferably a pore size between 75 nm and 300 nm, more preferably of 75 nm or 100 nm, most preferably of 100 nm.

6. The composition according to claim 5, wherein the selected fraction(s) are free or substantially free of viruses.

7. The composition according to any one of claims 1 to 6, wherein the blood serum or plasma is obtained from whole blood, preferably from venous blood.

8. The composition of any one of claims 1 to 7, wherein the blood serum or plasma is obtained from at least two, preferably more than four donors.

9. The composition according to any one of claims 1 to 8, wherein ultracentrifugation is performed at 100,000 to 120,000 × g for > 12 hours, preferably for 16 to 24 hours, more preferably at 100,000 × g for 24 hours.

10. Eye drops comprising the composition according to any one of claims 1 to 8.

11. A method of manufacture of eye drops comprising:
(i) providing blood serum or plasma, preferably wherein the blood serum or plasma is derived from more than two, preferably four donors;
(ii) fractionizing the blood serum or plasma via ultracentrifugation in a tube into a top fraction, one or more middle fraction(s) and a bottom fraction; preferably wherein ultracentrifugation is performed at 100,000 × g for 24 hours;
(iii) isolating one or more middle fraction(s), preferably the two middle fractions, wherein the isolated fraction(s) are **characterized by** being capable of inducing wound healing and/or having a protective effect against oxidative stress and/or reducing inflammatory response and/or decreasing excessive reactive oxygen species (ROS) activity in a human corneal epithelial cells-transformed (HCE-T) assay, and which are free or substantially free of lipids and lipoproteins;
(iv) optionally adding saline to the fraction(s);
(v) virus filtrating the fraction(s) or the fraction(s) mixed with saline, wherein the filter has a pore size between 15 nm to 80 nm, preferably between 15 nm to 40 nm, more preferably between 15 nm and 35 nm and even more preferably between 15 nm to 20 nm and most preferably 20 nm, preferably wherein a pre-filtration step is performed with a filter having a different pore size, preferably a pore size between 75 nm and 300 nm, more preferably of 75 nm or 100 nm, most preferably of 100 nm;
(vi) filling the fraction(s) or the fraction(s) mixed with saline of (v) into containers, preferably wherein the container is an ampule, preferably comprising a pump system; and optionally
(vii) freezing at -20°C.

12. Single doses or multiple doses container comprising the eye drops according to claim 10 or the eye drops manufactured according to claim 11.

13. The container according to claim 12, wherein the container is an ampule, preferably comprising a pump system.

14. The composition of any one of claims 1 to 9, or the eye drops of claim 10, or the eye drops as obtained by the method of claim 11 for use in treating, preventing and/or ameliorating damages on epithelial surfaces, preferably on non-keratinized epithelial surfaces, more preferably on non-keratinized epithelial surfaces of the eye, as well as eye diseases in which inflammatory processes and oxidative stress are recognized as underlying cause.

## Patentansprüche

1. Zusammensetzung, umfassend eine oder mehrere Blutfraktion(en), wobei die Fraktion(en) frei oder im Wesentlichen frei von Lipiden und Lipoproteinen ist/sind und durch ein Verfahren erhalten wurde(n), umfassend
(i) Fraktionierung von Blutserum oder -plasma durch Ultrazentrifugation in einem Röhrchen in eine obere Fraktion, eine oder mehrere mittlere Fraktion(en) und eine untere Fraktion; und
(ii) Auswählen einer oder mehrerer mittlerer Fraktion(en), wobei die ausgewählte(n) Fraktion(en) **dadurch gekennzeichnet** ist/sind, dass sie in der Lage ist/sind, Wundheilung zu induzieren und/oder eine schützende Wirkung gegen oxidativen Stress zu haben und/oder eine Entzündungsreaktion zu reduzieren und/oder eine übermäßige Aktivität reaktiver Sauerstoffspezies (ROS) in einem Assay mit menschlichen, transformierten Hornhautepithelzellen (HCE-T) zu verringern, wobei die Zusammensetzung dazu vorgesehen ist, topisch verabreicht zu werden, und vorzugsweise als Tropfen, Salbe oder Gel formuliert ist.

2. Zusammensetzung nach Anspruch 1, die ferner einen pharmazeutisch verträglichen Träger umfasst, wobei der Träger vorzugsweise keine Reizung des Auges hervorruft und vorzugsweise eine Salzlösung ist.

3. Zusammensetzung nach Anspruch 1 oder 2, wobei die ausgewählte(n) Fraktion(en) zwei Mittelfraktionen sind.

4. Zusammensetzung nach einem der Ansprüche 1 bis 3, wobei die ausgewählte(n) Fraktion(en) bei einer Konzentration von 1 % (v/v) einen Wundverschluss von etwa 70% des verwundeten Bereichs innerhalb von etwa 60-70 Stunden induzieren.

5. Zusammensetzung nach einem der Ansprüche 1 bis 4, wobei die ausgewählte(n) Fraktion(en) einer Filtration mit einem Filter mit einer Porengröße zwischen 15 nm und 80 nm, vorzugsweise zwischen 15 nm und 40 nm, besonders bevorzugt zwischen 15 nm und 35 nm und bevorzugter zwischen 15 nm und 20 nm und am meisten bevorzugt 20 nm unterzogen werden, wobei vorzugsweise ein Vorfiltrationsschritt mit einem Filter mit einer anderen Porengröße, vorzugsweise einer Porengröße zwischen 75 nm und 300 nm, besonders bevorzugt von 75 nm oder 100 nm, am meisten bevorzugt von 100 nm, durchgeführt wird.

6. Zusammensetzung nach Anspruch 5, wobei die ausgewählte(n) Fraktion(en) frei oder im Wesentlichen frei von Viren ist/sind.

7. Zusammensetzung nach einem der Ansprüche 1 bis 6, wobei das Blutserum oder - plasma aus Vollblut, vorzugsweise aus Venenblut, erhalten wird.

8. Zusammensetzung nach einem der Ansprüche 1 bis 7, wobei das Blutserum oder -plasma von mindestens zwei, vorzugsweise mehr als vier Spendern erhalten wird.

9. Zusammensetzung nach einem der Ansprüche 1 bis 8, wobei die Ultrazentrifugation bei 100.000 bis 120.000 × g für > 12 Stunden, vorzugsweise für 16 bis 24 Stunden, besonders bevorzugt bei 100.000 × g für 24 Stunden durchgeführt wird.

10. Augentropfen, die die Zusammensetzung nach einem der Ansprüche 1 bis 8 enthalten.

11. Verfahren zur Herstellung von Augentropfen, umfassend:
(i) Bereitstellen von Blutserum oder -plasma, wobei das Blutserum oder -plasma vorzugsweise von mehr als zwei, vorzugsweise vier Spendern stammt;
(ii) Fraktionieren des Blutserums oder -plasmas durch Ultrazentrifugation in einem Röhrchen in eine obere Fraktion, eine oder mehrere mittlere Fraktion(en) und eine untere Fraktion; wobei die Ultrazentrifugation vorzugsweise bei 100.000 × g für 24 Stunden durchgeführt wird;
(iii) Isolieren einer oder mehrerer Mittelfraktion(en), vorzugsweise der beiden Mittelfraktionen, wobei die isolierte(n) Fraktion(en) **dadurch gekennzeichnet** ist/sind, dass sie in der Lage ist/sind, Wundheilung zu induzieren und/oder eine schützende Wirkung gegen oxidativen Stress zu haben und/oder eine Entzündungsreaktion zu reduzieren und/oder eine übermäßige Aktivität reaktiver Sauerstoffspezies (ROS) in einem Assay mit menschlichen, transformierten Hornhautepithelzellen (HCE-T) zu verringern, und die frei oder im Wesentlichen frei von Lipiden und Lipoproteinen ist/sind;
(iv) gegebenenfalls Zugabe von Kochsalzlösung zu der/den Fraktion(en);
(v) Virusfiltration der Fraktion(en) oder der mit Kochsalzlösung gemischten Fraktion(en), wobei der Filter eine Porengröße zwischen 15 nm und 80 nm, vorzugsweise zwischen 15 nm und 40 nm, besonders bevorzugt zwischen 15 nm und 35 nm und noch bevorzugter zwischen 15 nm und 20 nm und am meisten bevorzugt 20 nm aufweist, wobei vorzugsweise ein Vorfiltrationsschritt mit einem Filter durchgeführt wird, der eine andere Porengröße aufweist, vorzugsweise eine Porengröße zwischen 75 nm und 300 nm, besonders bevorzugt von 75 nm oder 100 nm, am meisten bevorzugt von 100 nm;
(vi) Abfüllen der Fraktion(en) oder der mit Kochsalzlösung gemischten Fraktion(en) von (v) in Behälter, wobei der Behälter vorzugsweise eine Ampulle ist, die vorzugsweise ein Pumpsystem umfasst; und gegebenenfalls
(vii) Einfrieren bei -20°C.

12. Behälter für Einzeldosen oder Mehrfachdosen, der die Augentropfen nach Anspruch 10 oder die nach Anspruch 11 hergestellten Augentropfen enthält.

13. Behälter nach Anspruch 12, wobei der Behälter eine Ampulle ist, die vorzugsweise ein Pumpsystem umfasst.

14. Die Zusammensetzung nach einem der Ansprüche 1 bis 9 oder die Augentropfen nach Anspruch 10 oder die Augentropfen, wie sie durch das Verfahren nach Anspruch 11 erhalten werden, zur Verwendung bei der Behandlung, Vorbeugung und/oder Verbesserung von Schäden an Epithel oberflächen, vorzugsweise an nicht-keratinisierten Epithel oberflächen, besonders bevorzugt an nicht-keratinisierten Epitheloberflächen des Auges, sowie bei Augenkrankheiten, bei denen entzündliche Prozesse und oxidativer Stress als zugrundeliegende Ursache erkannt werden.

## Revendications

1. Composition comprenant une ou plusieurs fractions sanguines, dans laquelle ladite ou lesdites fractions sont exemptes ou sensiblement exemptes de lipides et de lipoprotéines et obtenue(s) par l'intermédiaire d'un procédé comprenant les étapes consistant à
(i) fractionner du sérum sanguin ou du plasma par ultracentrifugation dans un tube en une fraction supérieure, une ou plusieurs fractions médianes et une fraction inférieure ; et
(ii) (ii) sélectionner une ou plusieurs fractions médianes, dans laquelle la ou les fractions sélectionnées sont **caractérisées par** leur capacité à induire la cicatrisation des plaies et/ou à avoir un effet protecteur contre le stress oxydatif et/ou à réduire la réponse inflammatoire et/ou à diminuer les excès de activité des espèces réactives de l'oxygène (ROS) dans un test de transformation de cellules épithéliales cornéennes humaines (HCE-T),
dans laquelle composition est conçue pour être administrée par voie topique et est de préférence formulée sous forme de gouttes, de pommade ou de gel.

2. Composition selon la revendication 1, qui comprend en outre un véhicule pharmaceutique, de préférence dans laquelle le véhicule n'induit pas d'irritation de l'oeil et est de préférence salin.

3. Composition selon la revendication 1 ou 2, dans laquelle la ou les fractions sélectionnées sont deux fractions médianes.

4. Composition selon l'une quelconque des revendications 1 à 3, dans laquelle la ou les fractions sélectionnées à une concentration de 1 % (v/v) induisent la fermeture de la plaie d'environ 70 % de la zone de plaie en 60 à 70 heures environ.

5. Composition selon l'une quelconque des revendications 1 à 4, dans laquelle la ou les fractions sélectionnées sont soumises à une filtration avec un filtre ayant une taille de pore comprise entre 15 nm et 80 nm, de préférence entre 15 nm et 40 nm, de manière plus préférée entre 15 nm et 35 nm, et de manière encore plus préférée entre 15 nm et 20 nm, et de la manière la plus préférée de 20 nm, de préférence dans laquelle une étape de pré-filtration est réalisée avec un filtre ayant une taille de pore différente, de préférence une taille de pore comprise entre 75 nm et 300 nm, de manière plus préférée de 75 nm ou de 100 nm, de la manière la plus préférée de 100 nm.

6. Composition selon la revendication 5, dans laquelle la ou les fractions sélectionnées sont exemptes ou sensiblement exemptes de virus.

7. Composition selon l'une quelconque des revendications 1 à 6, dans laquelle le sérum sanguin ou le plasma est obtenu à partir de sang total, de préférence à partir de sang veineux.

8. Composition selon l'une quelconque des revendications 1 à 7, dans laquelle le sérum sanguin ou le plasma est obtenu à partir d'au moins deux, de préférence plus de quatre donneurs.

9. Composition selon l'une quelconque des revendications 1 à 8, dans laquelle l'ultracentrifugation est effectuée entre 100 000 et 120 000 × g pendant plus de 12 heures, de préférence pendant 16 à 24 heures, de manière plus préférée à 100 000 × g pendant 24 heures.

10. Collyre comprenant la composition selon l'une quelconque des revendications 1 à 8.

11. Procédé de fabrication de gouttes oculaires comprenant les étapes consistant à :
(i) fournir du sérum sanguin ou du plasma, dans lequel de préférence le sérum sanguin ou le plasma proviennent de plus de deux, de préférence de quatre donneurs ;
(ii) fractionner le sérum sanguin ou le plasma par ultracentrifugation dans un tube en une fraction supérieure, une ou plusieurs fractions médianes et une fraction inférieure ; de préférence dans lequel l'ultracentrifugation est effectuée à 100 000 × g pendant 24 heures ;
(iii) isoler une ou plusieurs fractions médianes, de préférence les deux fractions médianes, dans lequel la ou les fractions isolées sont **caractérisées par** leur capacité à induire la cicatrisation des plaies et/ou à avoir un effet protecteur contre le stress oxydatif et/ou à diminuer les excès de activité des espèces réactives de l'oxygène (ROS) dans un test de transformation de cellules épithéliales cornéennes humaines (HCE-T), et qui sont exemptes ou sensiblement exemptes de lipides et de lipoprotéines ;
(iv) facultativement ajouter une solution saline à la ou aux fractions ;
(v) filtrer en termes de virus la ou les fractions ou la ou les fractions mélangées à une solution saline, dans lequel le filtre présente une taille de pore comprise entre 15 nm et 80 nm, de préférence entre 15 nm et 40 nm, de manière plus préférée entre 15 nm et 35 nm, et de manière encore plus préférée entre 15 nm et 20 nm, et de la manière la plus préférée de 20 nm, de préférence dans lequel une étape de pré-filtration est réalisée avec un filtre ayant une taille de pore différente, de préférence une taille de pore comprise entre 75 nm et 300 nm, de manière plus préférée de 75 nm ou de 100 nm, de la manière la plus préférée de 100 nm ;
(vi) (vi) verser la ou les fractions ou la ou les fractions mélangées avec une solution saline de (V) dans des contenants, de préférence dans lequel le récipient est une ampoule, comprenant de préférence un système de pompe ; et facultativement
(vii) congeler à -20°C.

12. Contenant unidose ou multidose comprenant le collyre selon la revendication 10 ou le collyre fabriqué selon la revendication 11.

13. Contenant selon la revendication 12, dans lequel le contenant est une ampoule, comprenant de préférence un système de pompe.

14. Composition selon l'une quelconque des revendications 1 à 9, ou collyre selon la revendication 10, ou collyre obtenu par le procédé selon la revendication 11, destiné à être utilisé pour traiter, prévenir et/ou améliorer des dommages sur des surfaces épithéliales, de préférence sur des surfaces des surfaces épithéliales non kératinisées, de préférence sur des surfaces épithéliales non kératinisées de l'oeil, ainsi que des maladies oculaires dans lesquelles des processus inflammatoires et un stress oxydatif sont reconnus comme cause sous-jacente.
